# EUROPEAN PATENT SPECIFICATION

(11) **EP 2 708 641 B1**
(45) Date of publication and mention of the grant of the patent: **23.01.2019**
(21) Application number: 12731686.7
(22) Date of filing: 14.05.2012
(51) Int. Cl.: D06M 16/00, D06P 1/34, A61Q 5/06, D06P 5/15, D06P 5/20, D06M 10/02, D06P 5/22, D06M 11/34, A23L 5/43, A23L 29/00, A61K 8/97, A61Q 19/04, A61Q 5/10, A61K 8/66

(54) **METHOD FOR COLORING MATERIALS WITH NATURAL COLORANTS AND ITS ARTICLES**
VERFAHREN ZUM FÄRBEN VON MATERIALIEN MIT NATURFARBSTOFFEN UND DEREN ARTIKEL
PROCÉDÉ DE COLORATION DE MATIÈRES AVEC DES COLORANTS NATURELS ET ARTICLES CORRESPONDANTS

(30) Priority: 12.05.2011 PT 2011105698
(43) Date of publication of application: 19.03.2014
(73) Proprietor: Tintex - Textiles, S.A., 4920-009 Campos V.N. de Cerveira (PT)
(72) Inventor: DOS SANTOS MARINHO DA SILVA, Carla Joana, P-4715-343 Braga (PT); DA ROCHA CARDOSO, Ana Patrícia, P-4460-158 Custóias Mts (PT); ANTUNES BARROS, Alexandre António, P-4710-737 Braga (PT); MACHADO, Maria José, P-4760-152 Vila Nova de Famalicão (PT); DO CARMO CRISPIM RIBEIRO, Luís Filipe, P-4465 S. Mamede de Infesta Mts (PT); TARRIO AGREIRO BEZERRA, Rodrigo José, P-2005-473 Santarem (PT); MONTEIRO RODRIGUES, António José, P-2380-163 Alcanena (PT)
(74) Representative: Teixeira de Carvalho, Anabela
(86) International application number: PCT/IB2012/052395
(87) International publication number: WO 2012/153318

(56) References cited:
- WO-A1-2006/070402
- WO-A1-2011/089615
- DATABASE WPI Week 200318 Thomson Scientific, London, GB; AN 2003-178428 XP002683877, & JP 2002 266258 A (KURABO IND LTD) 18 September 2002 (2002-09-18)

## Description

### Technical field of the invention

The matter described in the present invention regards a process to prepare colored materials/substrates with good characteristics in terms of behavior and fastness, using only biodegradable agents naturally present in nature, by using an environmentally friendly process that is innocuous to the end user, since there is no generation of any recalcitrant or toxic residues.

The coloring process here described covers the use of vegetable extracts and enzymes, that will be used to generate color "in situ" into several substrates and the colored materials obtainable by the method described in the present invention. The materials therefore prepared can be used to produce several articles such as clothing, shoes, upholstery, coatings, home textiles, carpets, etc.

### Background of the invention

One of the most pollutant processes in the leather and textile industries is dyeing, a process that aims to provide color to a given substrate, being the objective, as a general rule, the permanent coloring of it. Besides the high consumption of water, energy and auxiliary chemicals (salts, tensioactive agents, mordant agents - i.e. a substance that is added to the dyeing bath with the specific function to keep the durability of the color, to be more resistant to the washings, detergents, bleaching agents and to solar exposure), the synthetic dyes that are used may persist longer in the environment and are not easily degraded (recalcitrant compounds), causing an environmental problem in sewage treatment systems, since they are very difficult to remove from the environment.

Accompanying the consumer's desire for more bright and stable colors, the synthetic dyes were produced to be more effective and therefore more visible and less biodegradable in the wastewater streams. The dye molecules were designed to be stable and to resist to the chemical, biological and luminous degradation, among others. The effluent treatment is therefore difficult and constitutes a serious environmental problem.

In this context, the development of less pollutant innovative coloration methods with good fastness properties is of paramount need for the textile and leather industries, mainly in the garment sector. Due to the growing environmental concerns, reflected in the more restrictive legal impositions and also in the progressive awareness of the business fabric about environmental issues, the search for cleaner technologies for processing leather and textiles that might prevent or mitigate the damages in the environment assumes a fundamental role.

Therefore, it is very important for the companies the investment on innovative processing systems for decreasing the environmental impact and raising substantially the added value of their products, by its quality and originality. This is the scope of the present invention that aims to apply innovative and ecological methods to several textile and leather substrates, among others, to provide them a given color.

One of the actual trends in the garment sector is the use of natural dyes extracted from the leaves, bark or pulp of several fruits or vegetables. Recent studies report the use of natural dyes to color several textile substrates, namely wool and cotton [Bechtold T, Mahmud-Ali A, Mussak R. 2007.Dyes and Pigments 75: 287-293].

One of the biggest obstacles to the use of these dyes is its low fastness to washing, light, friction and sweat (Natural Dyes. Edited by E. Perrin Akçakoca Kumbasar, ISBN 978-953-307-783-3, InTech, Published: November 14, 2011. DOI: 10.5772/1817).

Additionally, when natural dyes are used to dye natural substrates, an excessive amount of dyes, salts and mordent agents are used, to enable the coloring process. As example, to color cotton with natural dyes the process consists in placing the substrate into a solution at 70-100°C and pH 10-12, with 10 to 50% or more of the natural dye, 5-20 g/L of salt as well as mordent agents (aluminum, chromium, iron, among others). These mordent agents are extremely hazardous to the user and environment, besides increasing the dyeing process.

The document WO 2001/044563 reports the coloration by contacting the material with a mixture of aromatic diamine and one or more of a naphtol and an aminonaphthalene and an oxidation system comprising a hydrogen peroxide source and an enzyme exhibiting peroxidase activity or oxidase activity on one or more of the compounds of mixture. The material may be a fabric, yarn, fiber, garment or film made of fur, hide, leather, silk or wool, or made of diacetate, flax, linen, lyocel, polyacrilic, polyamide, polyester, ramie, rayon, triacetate or viscose. Although the cited document is from 2001, this technology was not implemented yet to the leather and textile industry. One of the major limitations is due to the low fastness of the cited dyeing process. The polymerization conditions have inevitably to be very well studied regarding the substrate, the natural compounds and the enzymes, so that the "in situ" polymerization generates the color permanently at the substrate surface. All the previous inventions ignored this fact, justifying the reporting of ineffective processes in what concerns the color fastness to use (washing, friction, sweat and light, for instance).

The document WO 2009/051569 describes a dyeing process for cotton and polyamide textile materials with indigo dye (C.I. Vat Blue 1) using an enzyme, reductase or oxidase, in the presence of a redox mediator, (example 1,8- dihydroxy-9,10-anthraquinone) or an oxidizing agent (example hydrogen peroxide), to promote the indigo reduction. The invention intends to decrease the high costs and negative environmental impact of the conventional dyeing processes for cellulose and polyamide fibers, with vat dyes, specifically indigo dyes.

The document US 2009/0226589 A1 describes a chemical process for producing more stable and resistant dyes, for coloring textile substrates, paper, leather, wood and cosmetics, using water soluble vegetable extracts modified by mixing with a vegetable mordent, a mimosa tannin. The described process includes the preparation of the natural dyes by plant extraction and mixture with the tannin, the homogenization of the solution and the dehydration of the condensed mixture. The obtained colors were yellow, orange, green, blue and red, being the objective of the inventors the replacement of anilines containing dyes, which are harmful to the environment and affect the health of the users and consumers. The authors state that the textile samples dyed with these dyes present a good penetration, good coloring yield and excellent fastness, although they do not present any values for these parameters.

The document WO 99/15137 reports the use of an enzymatic composition formulated in foam for coloring keratin based fibers such as hair, fur, leather and wool, comprising at least 1) an oxidative enzyme, typically an oxidoreductase selected from the group of laccases and enzymes related with oxidases and peroxidases, 2) at least a foaming agent, selected from the group of detergents and anionic surfactants, non-ionic, amphoteric and zwitterionic and 3) at least a precursor, selected from the group of diamines, aminophenols and phenols and ideally 4) at least one modifier, selected from the group of aromatic diamines, m-amino phenols and polyphenols.

The document also describes a method for coloring keratin fibers using the foam composition, which allows for a better uniformity of the color, with reduced damages on hair.

The document WO 99/17720 describes the use of an enzymatic composition for treating keratin fibers, preferentially dyeing, containing oxidoreductases with 2 electrons in the presence of a donor and at least an anionic surfactant.

The document WO 02/47633 reports an agent for coloring keratin fibers containing at least one compound with a nucleophilic reactive center, at least one alcohol from the group of aryl alcohols and benzyl alcohols derivatives and at least a proper oxidative enzyme.

The document WO 02/30371 describes a composition capable of providing a natural pigmentation and the method to achieve that composition, for coloring skin and/or keratin fibers. The composition comprises at least an enzyme with propigmentation activity, and a suitable amount of a catalytic system containing salts and Mn(II) and/or Zn(II) oxides and a second constituent from the group of alkaline hydrogen carbonates or alkaline earth and their mixtures.

In what concerns the leather dyeing, there are only inventions for coloring leather with synthetic dyes. As example, the document WO 2002/020897 describes a process for dyeing tanned leather with high intensity and excellent fastness, by using a process that consists in pre-treating the tanned leather in (a) an alkaline bath containing ammonia, primary amines or a mixture of ammonia and primary amines and then with a polyfunctional organic compound containing at least one aldehyde as functional group, or (b) in an acidic medium containing an organic polyfunctional compound and ammonia, primary amines or a mixture of ammonia and primary amines, and (c) dyeing in an alkaline medium with a water soluble dye containing at least a functional group capable of reacting with the functional group of the organic compound, establishing a covalent linkage.

The document WO 2010/034832 describes the dyeing of leather with monoazo dyes and its salts. According to the inventors, this formulation is excellent for dyeing leather in yellow with excellent fastness.

The document WO 2007/144280 describes tri-cationic dyes containing aromatic or hetero aromatic groups with a specific molecular formula.

The document WO2006070402 relates to a process of preparing garment comprising of: (i) selecting a suitable natural fiber/yarn/fabric, (ii) infusing color energy by dying the natural fiber/yarn/fabric using natural dyes, (iii) infusing the crystals power in the fabric, (iv) treating the said garment with the natural antimicrobial so as to obtain the desired fabric with infused color energy and crystal power.

The document JP2002266258 and Week 200318, Thomson Scientific, London, GB; AN 2003-178428 discloses two components based composition for dyeing a fabric wherein the composition comprises extracts of natural origin for coloring and an enzyme (oxidase; laccase).

The role of enzymes in leather processing is limited to processes for removing fur and fats, where proteases, lipases and amylases have an important job (for pilling and degreasing, for example).

### Summary of the invention

The present invention describes a method to provide color to several materials/substrates, using compounds naturally present on nature - natural colorants, which implies contacting the material with a mixture of vegetable extracts and enzymes with oxidase, oxidoreductase or peroxidase activity in the extracts and also the pre-treatment of the material by plasma and/or ozone. The materials/substrates can be fabrics, fibers, yarns, films or structures of natural or synthetic origin, such as, but not limited to: fur, skin, leather, hair, silk, wool, angora, cashmere, cotton, linen, jute, hemp, sisal, cork, lyocell, polyamide, polyester, acetate or viscose and its mixtures.

One of the problems that the present invention pretends to solve is describing a coloring process with natural dyes that allows for the penetration of the dye into the material and the durability - i.e., fastness of the color to the use (washing, friction, sweat, light, for instance).

This problem is solved by impregnating the material/substrate in a functional water bath and by previously activating the material. The activation can be performed by using plasma at atmospheric pressure and/or ozone, to generate specific functional groups at the surface, and therefore ensure an effective covalent linkage between the enzymatic precursors and all types of substrates. It was seen that the generation of functional groups at substrate surface, as polar groups, for instance hydroxyl or carboxyl groups, is fundamental to assure a covalent linkage of the natural precursors to the substrate and subsequently the fastness of the coloration. Better results on the fixation and fastness of the color of the material were also achieved when it was exposed to an additional step of pH change.

There isn't any process described in literature to color with fastness synthetic fibers or leather with natural dyes, without adding synthetic dyes and/or chemical agents like mordents and/or salts.

One embodiment of the present invention describes a method for coloring materials with natural dyes that comprises the following steps:
- prepare a water bath (functionalization bath) with extracts of natural origin for coloring and enzymes with oxidase or peroxidase - oxidoreductases - in the referred extracts, at temperatures between 30-60°C and pH between 4-6, preferentially 40-50°C and pH between 4 and 5;
- impregnate the material for coloring in the above-mentioned bath, where it can be added, in preferential cases, compounds for controlling the pH or buffers, as acetic acid, for example.

The enzyme stays active in the functionalization bath allowing its reuse for coloring different materials/substrates up to 5 times, which enables the decrease of the pollutant charge comparing with a conventional process for coloration/dyeing.

It is also described in this invention a post-treatment process for permanently fixating the color to the substrate, enabling excellent results of the colored substrates to fastness (to more than 30 washing cycles) and resistance (to friction, sweat, etc). The materials prepared according to the present invention are innocuous to the health or environment, having also a high degree of fastness, which gives them appropriate characteristics to several applications, ranging from furniture, hats, shoes, garments, upholstery, among others.

The present invention proposes an innovative method for coloring: the bio-coloration, using oxidoreductases. This class of enzymes promotes oxidation/reduction reactions that are capable of providing color to the substrates by using suitable enzymatic precursors.

In a preferred embodiment, the materials to be colored by the coloration method here described can be previously subjected to a pre-treatment process by plasma and/or ozone.

In a preferred embodiment, the impregnated material by the coloration method described by the present invention can be afterwards subjected to a step were pH is adjusted between 2 and 6. This post-treatment enables the deep fixation of the color to the substrate/material, to provide excellent results for fastness (keeping the color after more than 30 washing steps) and resistance (friction, sweat, etc). The materials prepared according to the present invention are innocuous to the health and environment, having also a high degree of fastness, which gives them appropriate characteristics to several applications, ranging from furniture, hats, shoes, garments, upholstery, among others.

In yet another preferred embodiment of the coloration method described in the present invention, the concentration of the mentioned enzyme can vary between 0.1-2000 mg/l; preferentially between 0.2-200 mg/l. More preferentially, the enzyme can be of vegetable, animal or microbial source; were the enzymes can be pyranose oxidases, glucose oxidases, glycerol oxidases, lactate oxidases, pyruvate oxidases, uricases, choline oxidases, sarcosine oxidases, bilirubin oxidases, laccases, tyrosinases, peroxidases, catalases, superoxide dismutases, or their mixtures. Inside the laccases group, better results were obtained for the fungal laccase or Aspergillus laccase.

The oxidoreductases promote in situ polymerization, and among this class we can find the laccases, which are enzymes that are capable of oxidizing polyphenolic compounds having as final acceptor of the electrons the oxygen. These enzymes can be found in plants, fungus and some bacteria.

The laccases appear as enzymes with better results on the coloration of materials/substrates with natural dyes namely leather and textile substrates, where colored materials with high fastness are obtained, by the proper linkage of the natural precursors and process conditions (temperature, time and pH), enabling the achievement of an extensive range of colors with good fastness.

In a preferred embodiment of the present method, the precursors used are compounds that are naturally present in nature such as diamines, aminophenols, phenols, polyphenols, or their mixtures. Still more preferentially, natural polyphenolic extracts such as caffeine, theine, vanilla, vegetable extracts such as mimosa, quebracho, pine, chestnut tree, ginger, Caesalpinia echinata or their mixtures. Since the process occurs in moderate temperature and pH conditions, does not use any pigment (synthetic compounds that are normally recalcitrant) using only natural compounds and enzymes, decreases the risk of allergic reactions. This innovative coloring method stands therefore as an ecologically sustainable process and attractive to the end user, due to its increasing awareness.

In yet another preferred embodiment of the coloring method described in the present invention, the mentioned natural extracts are diamines, aminophenols, phenols, polyphenols or their mixtures; preferentially natural polyphenolic extracts such as caffeine, theine, vanilla, vegetable extracts such as mimosa, quebracho, pine, chestnut tree, ginger, Caesalpinia echinata or their mixtures. The concentration of these vegetable extracts varies between 20-600 g/Kg o.w.f.- weight of fabric, more preferentially between 100-400 g/Kg o.w.f.

The coloring method described in the present invention can run from 30 minutes to 6 hours, preferentially from 1, 2, 3, 4, 5 hours, depending on the desired color, its intensity and the type of enzyme and natural agents used in the functionalization/impregnation bath.

The materials colored by the coloration method described in the present invention might be fur, skin, leather, hair, silk, wool, angora, cashmere, cotton, linen, jute, hemp, sisal, cork, lyocell, polyamide, polyester, polypropylene, polyurethane, acrylic, acetate, elastane, nylon or viscose and its mixtures.

The materials colored by the natural colorants obtainable by the coloring method described in the present invention show a color fastness of at least 4 in the grey scale, more preferentially between 4 and 5 in the grey scale. The materials might be synthetic or natural such as fur, skin, leather, hair, silk, wool, angora, cashmere, cotton, linen, jute, hemp, sisal, cork, lyocell, polyamide, polyester, polypropylene, polyurethane, acrylic, acetate, elastane, nylon or viscose and its mixtures. The determination of the color fastness is normalized and depends on the type of fastness considered (for example fastness to washing ISO 6330, fastness to friction ISO 12947, fastness to light ISO 105 and fastness to friction on dry and wet ISO 11640).

It is also a preferred embodiment of the present invention articles produced with the colored materials obtainable by the coloring method described in the present invention. These articles might be garments, belts, purses, hats or shoes.

### Detailed Description

The present invention describes a method for coloring - dyeing - materials with natural dyes, which involves:
- the contact of the material after the pre-treatment with a mixture of (a) vegetable extracts and (b) an enzyme with oxidase or peroxidase activity over at least one of the vegetable extracts contained in the mixture (a);
- and the pre-treatment of the material to be colored with plasma and/or ozone.

The materials/substrates might be fabrics, fibers, yarns, films or structures of natural or synthetic origin such as, but not limited to fur, skin, leather, hair, silk, wool, angora, cashmere, cotton, linen, jute, hemp, sisal, cork, lyocell, polyamide, polyester, polypropylene, polyurethane, acrylic, acetate, elastane, nylon or viscose and its mixtures.

The materials/substrates dyed or colored according to the present invention are innocuous to the health and environment, since only natural dyes are used, the process uses moderate temperature and pH conditions, less time and having also a high degree of fastness - fact that until this moment was not possible with natural dyes, which provides the final product with appropriate characteristics to several industrial applications, ranging from furniture, hats, shoes, garments, upholstery, among others.

Surprisingly, the described coloring process occurs at low temperatures and by periods of time lower than the conventional dyeing processes, where the simultaneous coloration of natural and synthetic substrates in a single step can be performed, which renders the process more profitable from the ecological and economical point of view.

### Figures description

For an easy understanding of the invention examples of preferred achievements of the invention will be described, which however do not intend to limit the object of the present invention, in which:
**Figure 1**: Exemplificative range of colors for samples of tanned leather (wet-white base), obtained by the bio-coloration process.
**Figure 2****:** Transversal cut of leather samples (optical microscope Leica DM 2500M): a) obtained by the coloring method described in this document over wet-white base; b) conventional dyeing over wet-blue base.
**Figure 3A****:** Graphic showing the influence of wool fiber diameter on K/S value, obtained by the coloring method described in this document.
**Figure 3B****:** Samples of bio-colored wool, with different fiber diameters (ordered by increasing diameter).
**Figure 4A****:** Force of rupture, obtained from the average strength - deformation curves for wool samples without and colored with the method described in this document.
**Figure 4B****:** Elongation at break obtained from the average strength - deformation curves for wool samples without and colored with the method described in this document.
**Figure 5****:** Graphic of color variation as a percentage of the initial K/S value, obtained for wool samples colored by the method described in the present invention, after reusing the coloring bath for 5 consecutive cycles.
**Figure 6****:** Graphic of color variation as a percentage of the initial K/S value, obtained for samples colored by the method described in the present invention, after several washing cycles, performed according to standard ISO 6330:2010.

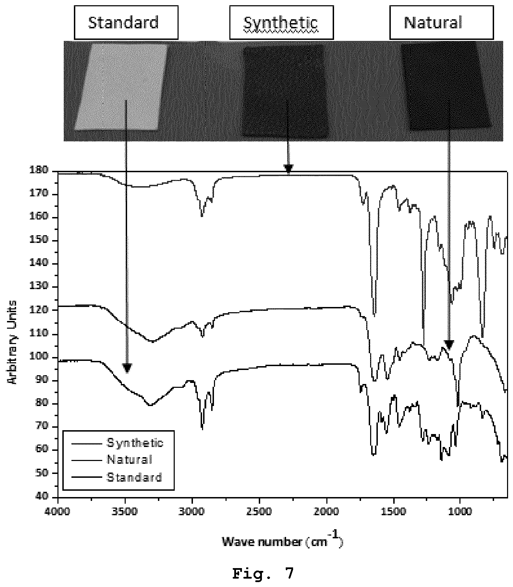

**Figure 7****:** Graphic of FTIR - Fourier transform infrared spectroscopy - showing the differences on leather surface for uncolored leather (standard), leather colored with a synthetic dye and leather colored by the method described in the present invention.

According to the present invention, several bio-colored substrates/materials are prepared, i.e. materials colored using only natural compounds and enzymes.

The preparation of the substrates comprises the following steps:
- Pre-treatment of the substrate using a plasmatic discharge, preferentially of chemical plasma using reactive gases, such as oxygen or nitrogen and/or to a stream of ozone, at room temperature;
- Preparation of the functionalization bath containing enzymes and colorants - agents of natural origin (such as diamines, aminophenols, phenols, polyphenols or its mixtures) suitable to provide the desired color;
- Reaction of the substrate impregnated in the functionalization bath, by exhaustion, in suitable pH, temperature and stirring conditions;
- Washing the substrates to remove the excess of natural agents not covalently bound;
- Post-treatment of the substrate by addition of a suitable amount of acid or alkali to attain a pH on the substrate surface between 2 and 6, more preferentially between 3 and 5 and still more preferentially between 4 and 5, for fixation of the color permanently to the substrate;
- Washing the substrates to remove the excess of acid or alkali, for neutralization.

In one embodiment of the present invention, the enzymes can be selected from the group of pyranose oxidases, glucose oxidases, glycerol oxidases, lactate oxidases, pyruvate oxidases, uricases, choline oxidases, sarcosine oxidases, bilirubin oxidases, laccases, tyrosinases, peroxidases, catalases, superoxide dismutases, or the vegetable or animal extracts containing the above-mentioned enzymes, in the presence of a suitable donor (or substrate) needed to the action of these enzymes.

The concentration of the used enzymes can vary between 0.1 and 2000 mg/L, preferentially between 0.2 e 200 mg/L, depending on its type and origin. Several agents of phenolic or polyphenolic base naturally present in nature were also used, such as caffeine, theine, vanilla, several vegetable extracts such as mimosa, quebracho, chestnut, pine, among other natural plant extracts. These agents are preferentially used in concentration between 20 to 600 g/Kg (o.w.f.), preferentially between 100 to 400 g/Kg (o.w.f.). The bath-ratio used in the exhaustion process might vary between 1:5 and 1:50 kg/L, and the range of temperatures from 30-60°C, preferentially from 40-50°C. The pH of the impregnation bath might have to be adjusted to a pH near 4.5-5, preferentially by adding acetic acid. Nevertheless, depending on the pre-treatment performed, this step can be omitted. The coloration process might run for 30 minutes to 6 hours, preferentially from 1, 2, 3, 4, 5 hours, depending on the desired color, its intensity and the type of enzyme and natural agents used in the functionalization or impregnation bath.

In another embodiment of this invention, the substrate/material might still be subjected to a final step for pH adjustment, by adding an acid or alkali, to permanently fixate the color to the substrate.

Therefore, the main objective of the present invention is to prepare bio-colored substrates by using an ecological process and using only compounds naturally present in nature. The materials prepared by using the present invention are innocuous to the health and the environment, having also a high degree of fastness, which gives them characteristics suitable to several applications, ranging from garments to shoes, furniture, and upholstery, among others.

### Preferred examples of application

The following examples are given only to provide a better understanding of the matter, can be combined between them, and they should not be considered limitative in the scope of the present invention.

### Example 1

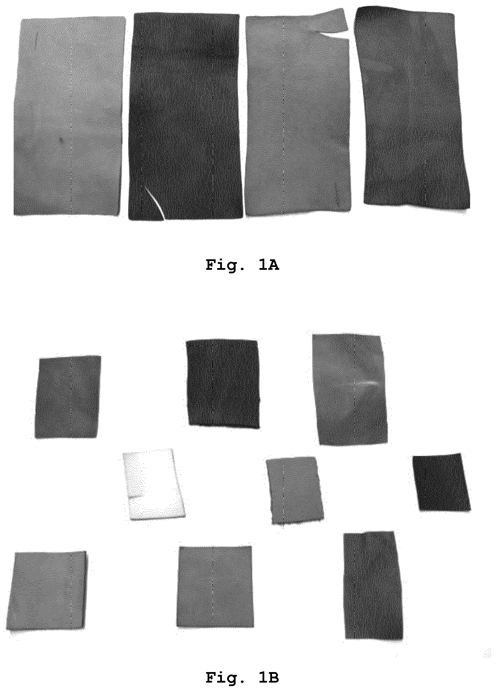

A given amount of goat skin and/or mixed breeds with wet-white tanning was introduced into a closed vessel and subjected to an ozone stream of 2.8 g/h for 30 minutes. After this time the bio-coloration process was performed, by adding 2% (o.w.f.) of a commercial fungal laccase, 11% (o.w.f.) of natural extract of Caesalpinia echinata (Brazilian tree known as Pau-Brasil) and 2% of ginger extract (Zingiber officinale). These conditions were chosen to attain black bio-colored leather, and the process was performed for 30 min, 40°C in a foulon. The color of the leather is given by the appropriate choice of the enzyme and the vegetable extracts, where an extensive range of colors can be obtained (see figure 1).

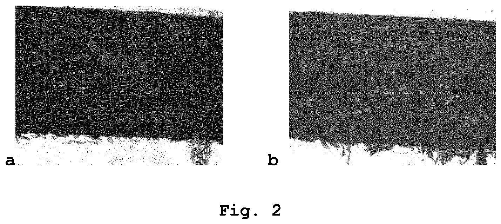

After the bio-coloration process, a post-treatment process with formic acid was performed, to ensure the deep fixation of the natural agents to the bio-colored skin, and the leather was washed. The visualization of the transversal cut (see figure 2) allow us to verify that the bio-coloring process was effective, since the color penetrated inside all the leather thickness (Figure 2.a) and showing a better performance than a wet-blue tanned leather dyed with a synthetic dye (Figure 2.b). One of the most important factors for coloring substrates is the deepness of the coloration, which means, the degree of penetration of the color along all the thickness of the substrate. Initially it was thought that the bio-coloration would be only superficial. Nevertheless, it was possible to verify an excellent capacity for the color penetration along all the substrate thickness, showing also a superior uniformity of the color than the normally obtained using a conventional process, as can be seen in figure 2.

To validate its characteristics, the bio-colored skins were evaluated for the color fastness to sweat, friction (dry and wet) and resistance of the hair side to cracks and rupture, a test normally called "lastomer". It was decided to include also the analysis of fastness to light, an important parameter in the case of bio-colored samples with natural mediators.

Concerning the fastness to friction, the tests were performed according to standard ISO 11640 (friction to dry and wet), were a minimum result of 4 in the grey scale is expected. The obtained results were the following:
- Friction Veslic - hair/degree of staining - dry (100 cycles): 5;
- Friction Veslic - hair/degree of staining - wet (50 cycles): 4.
These results are excellent, since 5 is the maximum possible value, which means that no staining occurred.

In what concerns the color fastness to sweat, this parameter was tested according to standard ISO 11641, in the hair side, to evaluate the degree of staining with different types of textile fibers. The result was satisfactory to all tested fibers, namely acrylic, cotton, lyocell, nylon 6.6 and polyester (4 in the grey scale), and the worst result was obtained for wool (3/4 in the grey scale) .
The result obtained for light fastness, determined according to standard ISO 105-B02 (xenon lamp), a critical parameter for bio-coloration with natural mediators, was equal to 4 (in the blue scale), being considered an excellent result.

Additionally, physical-mechanical assays were conducted, to determine the tear resistance (according to standard ISO 3377-2:2002) and the stretching and traction resistance - "lastomer" (according to standard ISO 3376:2002). The obtained values show that the bio-coloration process does not affect significantly the mechanical properties of the skin. In the bio-coloration process with vegetable extracts it was predicted a decrease in the mechanical resistance of the skin, due to the fact that a high dosage of this type of products might generate an overload, decreasing the lubrication of the fibrous structure of the skin.

The black bio-colored leather was also analyzed in what concerns the heavy-metals (aluminum, barium, lead, cobalt and chromium) aromatic amines, formaldehyde, free formaldehyde, trichlorophenol, tetrachlorophenol and pentachloro phenol detection. These chemical species were not detected, or are only present in trace amount, which means that the bio-coloration process allows for attaining leather free from harmful substances, with a decreased risk for creating allergic reactions and more appealing to the end-user, with increasing awareness and exigency.
- Amount of heavy metals:
   [Barium] < 5 ppm;
   [Cobalt] < 5 ppm;
   [Lead] < 5 ppm;
   [Aluminum] < 100 ppm;
   [Chromium] < 500 ppm.
- Free from aromatic amines (amount < quantification limit
- 30 ppm)
- Free from pentachlorophenol (amount < quantification limit - 5 ppm)
- Free from tetrachlorophenol (amount < quantification limit - 0.1 ppm)
- Free from trichlorophenol (amount < quantification limit
- 5 ppm)
- Amount of formaldehyde < 50 ppm
- Resistance to Veslic friction (dry or wet) > 3-4
- Resistance of the hair side to extension - lastomer > 7.0 mm
- Resistance to traction > 20 N/mm2
- Resistance to tearing > 20 N (thickness < 1.0 mm)

### Example 2

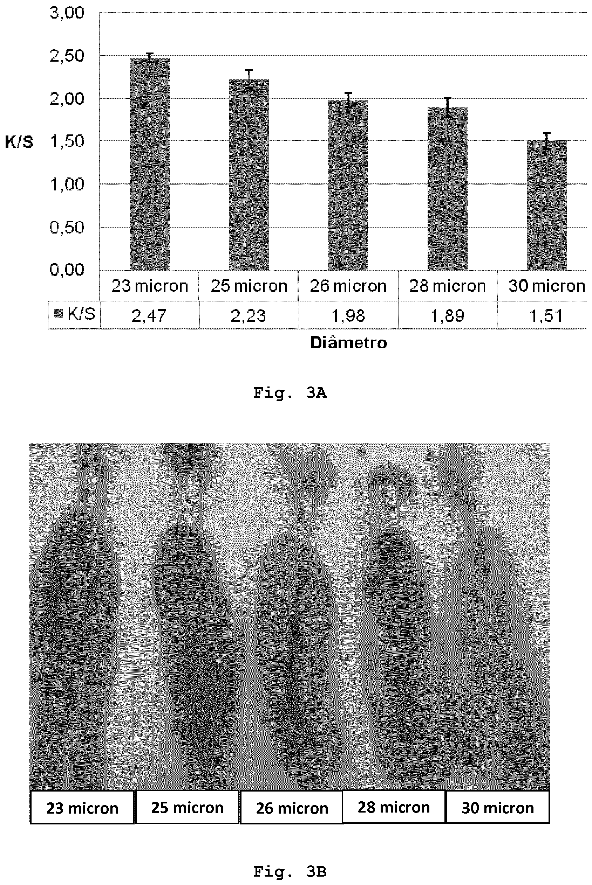

A given amount of merino wool (23pm of medium fiber diameter) was introduced in a plasma reactor at atmospheric pressure, and subjected to a plasma discharge in the following conditions: speed of 10m/min, reactive gas oxygen at 3% in a stream of Argon and power of the discharge of 9 kW.
After this process, the bio-coloration began by adding 20 mg/L of a laccase from Aspergillus and 20 g/L of coffee, being the conditions chosen to attain a brown wool fabric. It was seen that the penetration inside wool was inversely proportional to the fiber diameter (figure 3), since that the increase in the number of cuticle layers in the fiber is imposing a barrier to the penetration of the natural agents, and therefore the coloration is being more superficial. The process was performed at 40°C, for a period of 4 hours, in a water bath with a speed of 150 rpm. After the bio-coloration process, trichloroacetic acid was added to ensure the fixation of the natural agents used.

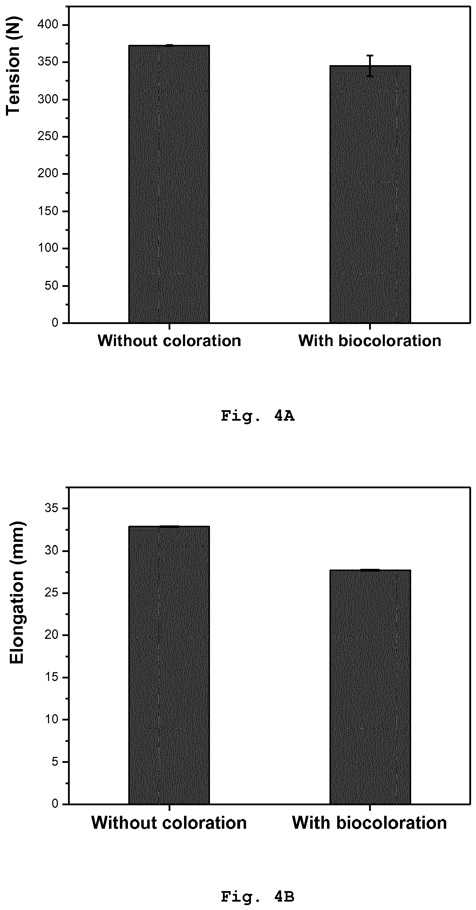

To validate its characteristics, the bio-colored wool was analyzed in what concerns the color fatness to sweat, friction (dry and wet), resistance to tearing in an Instrom (see figure 4) and visual penetration of the color by optical microscopy. It was seen that the bio-colored wool does not lose any resistance in the process, by comparing with the control (figure 4), having also excellent results for the color fastness.

### Example 3

A given amount of polyamide was introduced into a plasma reactor at atmospheric pressure and subjected to a plasma discharge in the following conditions: speed of 10m/min, reactive gas nitrogen at 3% in a stream of Argon and power of the discharge of 9 kW.
After this time, the bio-coloration process was performed by adding 50 mg/L of a laccase from Aspergillus, 10 g/L of natural extract from Caesalpinia echinata (Brazilian tree known as Pau-Brasil) and 10 g/L of mimosa and quebracho extracts, being the conditions chosen to attain an orange polyamide fabric. The process was performed for 2 hours at 50°C, in a water bath with 150 rpm of stirring. After the bio-coloration process, acetic acid was added to ensure the fixation of the natural agents used in the bio-coloring process.

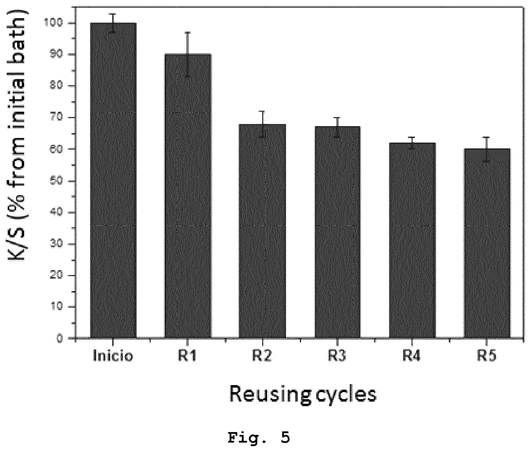

The functionalization bath was reused for 5 consecutive cycles, without adding any component, and a good performance of the fabric coloration was obtained, as can be seen in figure 5. This example demonstrates that it is possible to reuse the coloring bath up to 5 cycles, without significant losses of the color intensity, showing an excellent homogeneity.

To validate its characteristics, the bio-colored polyamide was analyzed for its color fastness to washing and water in a certified reference laboratory:
Fastness of the color to water (ISO 105 E01:1994) - color change 4-5
Fastness of the color to washing (ISO 105 C06:1994) - color change 4-5

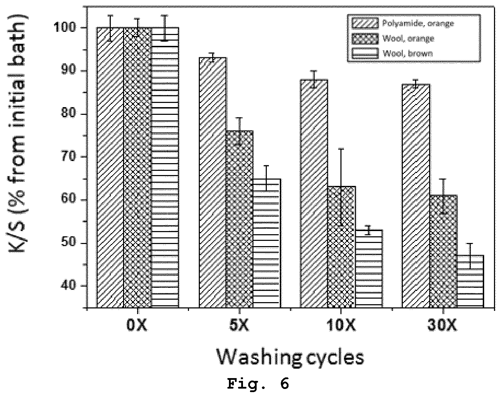

It was also analyzed the fastness of the color after 30 washing cycles, performed according to standard ISO 6330:2010 (see figure 6). The results demonstrate that after the first 5 cycles the polyamide orange color remains practically unchanged (in terms of percentage of the initial K/S). For the wool colored in the same bath, the fastness was not so satisfactory, demonstrating the importance of using a suitable enzyme and vegetable extracts for each substrate and color being studied.

### Example 4

A given amount of goat skin and/or of mixed breeds with wet-white tanning was introduced into a closed vessel and subjected to an ozone stream of 2.8 g/h for 30 minutes. After this time the bio-coloration process was performed, by adding 2% (o.w.f.) of a commercial fungal laccase, 11% (o.w.f.) of natural extract of Caesalpinia echinata (Brazilian tree known as Pau-Brasil) and 2% of ginger extract (Zingiber officinale). These conditions were chosen to attain black bio-colored leather, and the process was performed for 30 min, 40°C in a foulon. Simultaneously, the same leather was dyed with a conventional process of the leather industry, using a synthetic dye to provide the black color.

After the coloring process, the samples were screened to try to identify the dye used (natural vs synthetic). Figure 6 highlights the differences found in the chemical characterization of the leather by Fourier transform infrared spectroscopy with total attenuated reflectance (FTIR/ATR). The synthetic dye has differentiated bands, from the standard and bio-colored leather, allowing the detection of the coloring process.

Therefore, the method described in the present invention allows attaining also a screening process for color tracking, to verify the type of dye used (natural vs synthetic). The bio-coloring process can be tracked by using chemical analysis techniques, which might be used separately or combined to identify the dyes, such as UV-vis spectroscopy and Fourier transform infrared spectroscopy (FTIR), chromatography, mass spectrometry and Magnetic resonance imaging (MRI). These techniques might be used directly on the colored substrates or after the extraction of the dyes with proper solvents. Additionally, the monitoring of the bio-coloring process by the method described in the present invention is possible, when compared with other coloring methods using natural dyes, since that for the first time it is described a coloring process with high levels of color fastness for synthetic fibers using only natural based dyes and enzymes. If the chromatographic profile found reveals that the dye used was a natural one, a simple fastness to washing analysis allows detecting if the regarded substrate was colored with the method described in the present invention or other process using natural dyes.

The present invention is not, naturally, and by any means restricted to the examples described in this document and an average skilled person in the art might foreseen several possibilities of changing it without diverging from the general idea of the invention, as defined in the claims.

The preferred examples described above are obviously combinable among them. The following claims define additionally preferred embodiments of the present invention.

## Claims

1. Method for coloring materials with natural dyes comprising the following steps:
• preparing a water bath comprising extracts of natural origin for coloring and enzymes with oxidase or peroxidase activity in the referred extracts, at a temperature between 30-60 °C and a pH between 4-6;
• impregnating the material for coloring in the aforementioned bath;
wherein the materials are previously subjected to a pre-treatment process with plasma and/or ozone.

2. Method, according to previous claims, in which the coloring temperature is 40ºC and the pH is between 4 and 5.

3. Method, according to previous claims, in which the impregnated material is subsequently subjected to a step of pH adjustment at the surface of the referred material, between 2 and 6.

4. Method, according to previous claims, in which the concentration of the referred enzyme varies between 0.1-2000 mg/l.

5. Method, according to the previous claim, in which the concentration of the referred enzyme varies between 0.2-200 mg/l.

6. Method, according to previous claims, in which the referred enzymes are from vegetable, animal or microbial source preferably the referred enzymes are pyranose oxidases, glucose oxidases, glycerol oxidases, lactate oxidases, pyruvate oxidases, uricases, choline oxidases, sarcosine oxidases, bilirubin oxidases, laccases, tyrosinases, peroxidases, catalases, superoxide dismutases, or their mixtures.

7. Method, according to the previous claim, in which the referred laccases are fungal laccase, laccase from Aspergillus.

8. Method, according to previous claims, in which the concentration of the referred extracts from vegetable origin varies between 20-600 g/Kg o.w.f, preferably 100-400 g/Kg o.w.f.

9. Method, according to previous claims, in which the referred natural extracts are diamines, aminophenols, phenols, polyphenols or their mixtures.

10. Method, according to the previous claim, in which the referred natural polyphenolic extracts are caffeine, theine, vanilla, several vegetable extracts such as mimosa, quebracho, chestnut, pine, ginger, *Caesalpinia echinata* or their mixtures.

11. Method, according to previous claims, in which the referred materials to be colored are fur, skin, leather, hair, silk, wool, angora, cashmere, cotton, linen, jute, hemp, sisal, cork, lyocell, polyamide, polyester, polypropylene, polyurethane, acrylic, acetate, elastane, nylon or viscose and its mixtures.

12. Materials that are colored by natural colorants obtainable by the process described in any of the previous claims in which the color has a fastness of at least 4 in the grey scale measured according to standard ISO 11640.

13. Materials, according to the previous claim, in which the referred materials are synthetic or natural preferably said materials are fur, skin, leather, hair, silk, wool, angora, cashmere, cotton, linen, jute, hemp, sisal, cork, lyocell, polyamide, polyester, polypropylene, polyurethane, acrylic, acetate, elastane, nylon or viscose and its mixtures.

14. Articles comprising the colored materials according to the claims 12-13.

15. Articles according to the previous claim, in which the articles are garments, wallets, purses, shoes, hats, belts and upholstery, among others.

## Patentansprüche

1. Verfahren zum Färben von Materialien mit natürlichen Färbemitteln, dass es die folgende Schritte umfasst:
• vorbereiten eines Wasserbads mit Extrakten natürlichen Ursprungs zum Färben und Enzymen mit Oxidase- oder Peroxidase-Aktivität in besagten Extrakten bei einer Temperatur zwischen 30-60 °C und einem pH-Wert zwischen 4-6;
• imprägnieren des zu färbenden Materials in dem zuvor genannten Bad;
wobei die Materialien zuvor einer Vorbehandlung mit Plasma und/oder Ozon unterzogen werden.

2. Verfahren gemäß den vorherigen Ansprüchen, bei dem die Färbetemperatur 40°C beträgt und der pH-Wert zwischen 4 und 5 liegt.

3. Verfahren gemäß den vorherigen Ansprüchen, bei dem das zu imprägnierende Material anschließend einem Schritt zur pH-Anpassung auf der Oberfläche des besagten Materials zwischen 2 und 6 unterzogen wird.

4. Verfahren gemäß den vorherigen Ansprüchen, bei dem die Konzentration der besagten Enzyme zwischen 0,1-2000 mg/l variiert.

5. Verfahren gemäß den vorherigen Ansprüchen, bei dem die Konzentration der besagten Enzyme zwischen 0,2-200 mg/l variiert.

6. Verfahren gemäß den vorherigen Ansprüchen, bei dem die besagten Enzyme von einer pflanzlichen, tierischen oder mikrobiellen Quelle stammen, wobei die besagten Enzyme vorzugsweise Pyranose-Oxidasen, Glucose-Oxidasen, Glycerin-Oxidasen, Laktat-Oxidasen, Pyruvat-Oxidasen, Uricasen, Choline-Oxidasen, Sarkosin-Oxidasen, Bilirubin-Oxidasen, Laccasen, Tyrosinase, Peroxidase; Katalasen, Hyperoxid-Dismutasen oder Mischungen davon.

7. Verfahren gemäß dem vorherigen Anspruch, bei dem die besagten Laccasen aus Pilzen, Laccassen aus Aspergillus sind.

8. Verfahren gemäß den vorherigen Ansprüchen, bei dem die Konzentration der besagten Extrakte pflanzlichen Ursprungs zwischen 20-600 g/kg o.w.f. variieren, vorzugsweise zwischen 100-400 g/kg o.w.f.

9. Verfahren gemäß den vorherigen Ansprüchen, bei dem die besagten natürlichen Extrakte Diamine, Aminophenole, Phenole, Polyphenole oder Mischungen davon.

10. Verfahren gemäß den vorherigen Ansprüchen, bei dem die besagten natürlichen polyphenolischen Extrakte Koffeine, Thein, Vanille, verschiedene pflanzliche Extrakte wie Mimose, Quebracho, Rosskastanie, Kiefer, Ingwer, Caesalpinia echinata oder Mischungen davon.

11. Verfahren gemäß den vorherigen Ansprüchen, bei dem die besagten zu färbenden Materialien Fell, Haut, Leder, Haar, Seide, Wolle, Angora, Kaschmir, Baumwolle, Leinen, Jute, Hanf, Sisal, Kork, Lyocell, Polyamid, Polyester, Polypropylen, Polyurethan; Acryl, Azetat, Elastan, Nylon oder Viskose oder Mischungen davon.

12. Materialien, die durch natürliche Färbemittel gefärbt wurden, die über ein in allen vorherigen Verfahren beschriebenes Verfahren gewonnen wurden, bei dem die Farbe eine Beständigkeit von mindestens 4 auf der nach ISO Norm 11640 gemessenen Skala aufweist.

13. Materialien gemäß dem vorherigen Anspruch, bei denen die besagten zu färbenden Materialien natürlich oder synthetisch sind, wobei besagte Materialien bevorzugt Fell, Haut, Leder, Haar, Seide, Wolle, Angora, Kaschmir, Baumwolle, Leinen, Jute, Hanf, Sisal, Kork, Lyocell, Polyamid, Polyester, Polypropylen, Polyurethan; Acryl, Azetat, Elastan, Nylon oder Viskose oder Mischungen davon.

14. Artikel, die gefärbte Materialien gemäß den Ansprüchen 12-13 enthalten.

15. Artikel gemäß dem vorherigen Anspruch, bei denen die Artikel unter anderem Kleidungsstücke, Geldbeutel, Handtaschen, Schuhe, Hüte, Gürtel, Polsterungen sind.

## Revendications

1. Méthode pour teindre des matériaux avec des teintures naturelles comprend les étapes suivantes :
• préparer un bain-marie comprenant des extraits d'origine naturelle pour teindre et des enzymes à activité d'oxydase ou de peroxydase dans lesdits extraits, à une température située entre 30-60ºC et un pH situé entre 4-6 ;
• imprégner le matériau à teindre dans le bain supramentionné ;
dans laquelle les matériaux sont préalablement soumis à un processus de pré-traitement au plasma ou à l'ozone.

2. Méthode selon les revendications antérieures, dans laquelle la température de teinture est de 40ºC et le pH est situé entre 4 et 5.

3. Méthode, selon les revendications antérieures, dans laquelle le matérieu imprégné est par la suite soumis à une étape d'ajustement de pH entre 2 et 6 sur la surface dudit matériau.

4. Méthode selon les revendications antérieures, dans laquelle la concentration de ladite enzyme varie entre 0,1-2000 mg/l.

5. Méthode, selon la revendication précédente, dans laquelle la concentration de ladite enzyme varie entre 0,2-200 mg/l.

6. Méthode, selon les revendications antérieures, dans laquelle lesdites enzymes sont d'origine végétale, animale ou microbienne lesdites enzymes étant de préférence des pyranose oxidases, glucose oxydases, glycerol oxydases, lactate oxydases, pyruvate oxydases, uricases, choline oxydases, sarcosine oxydases, bilirubine oxydases, laccases, tyrosinases, peroxydases, catalases, superoxydes dismutases, ou un mélange de ceux-ci.

7. Méthode, selon la revendication précédente, dans laquelle lesdites laccases sont une laccase fongique, laccase d'Aspergillus.

8. Méthode, selon les revendications antérieures, dans laquelle la concentration desdits extraits d'origine végétale varie entre 20-600 g/Kg o.w.f (de l'anglais « *Weight of Fabric* »), de préférence 100-400 g/Kg o.w.f.

9. Méthode, selon les revendications antérieures, dans laquelle lesdits extraits naturels sont des diamines, aminophénols, phénols, polyphénols, ou un mélange de ceux-ci.

10. Méthode, selon la revendication précédente, dans laquelle lesdits extraits polyphénoliques naturels sont la caféine, la théine, la vanille, plusieurs extraits végétaux tels que le mimosa, le quebracho, la châtaigne, le pin, le gingembre, le caesalpinia echinata ou un mélange de ceux-ci.

11. Méthode, selon les revendications antérieures, dans laquelle lesdits matériaux devant être teints sont de la fourrure, de la peau, du cuir, des poils, de la soie, de la laine, de l'angora, du cachemire, du coton, du lin, de la jute, du chanvre, du sisal, du liège, du lyocell, du polyamide, du polyester, du polypropylène, du polyuréthane, de l'acrylique, de l'acétate, de l'élasthane, du nylon ou de la viscose et ses mélanges.

12. Matériaux qui sont teints par des teintures naturelles obtenues à travers le processus décrit dans l'une quelconque des revendications antérieures dans lesquelles la couleur dispose d'une stabilité d'au moins 4 dans l'échelle de gris mesurée d'après le standard ISO 11640.

13. Matériaux, selon la revendication précédente, dans laquelle lesdits matériaux sont synthétiques ou naturels, lesdit matériaux étant de préférence de la fourrure, de la peau, du cuir, des poils, de la soie, de la laine, de l'angora, du cachemire, du coton, du lin, de la jute, du chanvre, du sisal, du liège, du lyocell, du polyamide, du polyester, du polypropylène, du polyuréthane, de l'acrylique, de l'acétate, de l'élasthane, du nylon ou de la viscose et ses mélanges.

14. Articles comprenant les matériaux teints selon les revendications 12-13.

15. Articles selon la revendication précédente, dans laquelle les articles sont des vêtements, des portefeuilles, des sacs-à-main, des chaussures, des chapeaux, des ceintures et des tissus d'ameublement, entre autres.
